(19) Europäisches Patentamt
European Patent Office
Office européen des brevets

(11) Veröffentlichungsnummer: **0 439 426 A1**

(12) **EUROPÄISCHE PATENTANMELDUNG**

(21) Anmeldenummer: **91810029.8**

(22) Anmeldetag: **15.01.91**

(51) Int. Cl.⁵: **A01N 37/44**, C07C 229/48, C07C 229/76

(30) Priorität: **24.01.90 CH 228/90**

(43) Veröffentlichungstag der Anmeldung: **31.07.91 Patentblatt 91/31**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel (CH)**
Anmelder: **Degussa Aktiengesellschaft**
**Weissfrauenstrasse 9**
**W-6000 Frankfurt am Main 1 (DE)**

(72) Erfinder: **Ackermann, Peter, Dr.**
**Hangelimattstrasse 113**
**CH-4148 Pfeffingen (CH)**
Erfinder: **Fischer, Hanspeter, Dr.**
**Burggartenstrasse 14**
**CH-4103 Bottmingen (CH)**
Erfinder: **Vogel, Rolf, Dr.**
**Aumattstrasse 109**
**CH-4153 Reinach (CH)**
Erfinder: **Drauz, Karlheinz, Dr.**
**Zur Marienruhe 13**
**W-6463 Freigericht 1 (DE)**
Erfinder: **Hasseberg, Hans-Albrecht, Dr.**
**Kastanienstrasse 16**
**W-6458 Rodenbach (DE)**
Erfinder: **Hasselbach, Hans-Jochen, Dr.**
**Deutschordenstrasse 6**
**W-6460 Gelnhausen (DE)**
Erfinder: **Knaup, Günter, Dr.**
**Friedhofstrasse 8**
**W-6454 Bruchköbel (DE)**
Erfinder: **Krimmer, Hans-Peter, Dr.**
**Am Weissen Turm 48**
**W-6000 Frankfurt 60 (DE)**
Erfinder: **Schäfer, Matthias, Dr.**
**Siegfriedstrasse 34**
**W-8753 Obernburg (DE)**

(54) **Mikrobizide Mittel.**

(57) 1-Aminocyclohexancarbonsäure-Derivate der Formel I

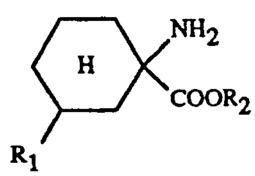

(I)

oder deren Säureadditionssalze oder Metallkomplexe lassen sich zumSchutz von Pflanzen vor Mikroorganismen einsetzen. In der Formel I bedeutetet $R_1$ Wasserstoff oder $C_1$-$C_4$-Alkyl und $R_2$ Wasserstoff, $C_1$-$C_6$-Alkyl, Benzyl, oder einen unsubstituierten oder durch Kohlenwasserstoffreste mit maximal 20 C-Atomen substituierten Ammoniumrest oder ein Metallionenäquivalent.

Verbindungen der Formel I lassen sich in Form von Pflanzenschutzmitteln mit geeigneten Trägermaterialien einsetzen.

EP 0 439 426 A1

# MIKROBIZIDE MITTEL

Die vorliegende Erfindung betrifft Mittel zum Bekämpfen von pflanzenpathogenen Pilzen und zur Verhütung von Pilzbefall, und ist dadurch gekennzeichnet, dass diese als Wirkstoff mindestens ein 1-Aminocyclohexancarbonsäure-Derivat der Formel I

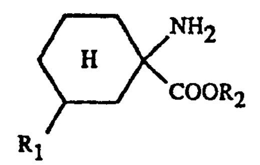

(I)

oder dessen Säureadditionssalze oder einen Metallkomplex von diesem enthalten, wobei $R_1$ Wasserstoff oder $C_1$-$C_4$-Alkyl bedeutet, und $R_2$ Wasserstoff, $C_1$-$C_6$-Alkyl, Benzyl, oder einen unsubstituierten oder durch Kohlenwasserstoffreste mit maximal 20 C-Atomen substituierten Ammoniumrest oder ein Metallionenäquivalent darstellt, zusammen mit einem pflanzenphysiologisch unbedenklichen Trägermaterial und gegebenenfalls weiteren Verteilungsmitteln. Die Erfindung betrifft weiterhin die entsprechende Verwendung der Verbindungen der Formel I zum Schutz von Pflanzen, Pflanzenteilen oder zum Schutz des Vermehrungsguts der Pflanzen, insbesondere des Saatguts.

Unter Alkyl soll, je nach Kettenlänge, hier und im folgenden Methyl, Ethyl, Propyl, Butyl, Pentyl, Hexyl sowie ihre jeweiligen Isomeren (z.B. Isopropyl, sek. Butyl, tert. Butyl, Isoamyl, Neopentyl usw.) verstanden werden.

Beispiele salzbildender Säuren sind anorganische Säuren (Halogenwasserstoffsäuren wie Chlorwasserstoffsäure, Bromwasserstoffsäure, Jodwasserstoffsäure sowie Schwefelsäure, Salpetersäure oder Phosphorsäure) und organische Säuren wie Essigsäure, Difluorchloressigsäure, Trifluoressigsäure, Trichloressigsäure, Propionsäure, Glykolsäure, Thiocyansäure, Maleinsäure, Apfelsäure, Milchsäure, Bernsteinsäure, Zitronensäure, Benzoesäure, Zimtsäure, Oxalsäure, Terephthalsäure, Ameisensäure, Benzolsulfonsäure, p-Toluolsulfonsäure, Methansulfonsäure, Salicylsäure, Mandelsäure, p-Aminosalicylsäure, 2-Phenoxybenzoesäure, Pyridin-3-sulfonsäure, Nicotinsäure oder 2-Acetoxybenzoesäure.

Metallkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der ersten bis vierten Hauptgruppe wie Na, Ca, Mg, Aluminium, Zinn oder Blei sowie der Elemente der ersten bis achten Nebengruppen wie Chrom, Mangan, Eisen, Kobalt, Nickel, Kupfer, Zink, Silber usw. vorzugsweise der Elemente der ersten, zweiten sowie sechsten bis achten Nebengruppen. Dabei können die Metalle in den verschiedenen ihnen zukommenden Wertigkeiten vorliegen. Die Metallkomplexe der Formel I können ein oder mehrere organische Molekülanteile enthalten.

Metallsalze der Formel I bestehen aus dem zugrundeliegenden Säureanion der cyclischen Aminocarbonsäure und einem Metallkation, oder mehreren verschiedenen, z.B. $Na^+/Fe^{3+}$ ; $Na^+/K^+$ ; $Ca^{++}/Mg^{++}$ ; $Cu^{++}/Ni^{++}/Mn^{++}/Co^{++}$ etc.

Salze aliphatischer Amine der Formel I enthalten das zugrundeliegende Säureanion der cyclischen Aminocarbonsäure und das Ammoniumion eines Amins, z.B. $N(Alkyl)_3$, $NH(Alkyl)_2$, $NH_2(Alkyl)$ wie $N(CH_3)_3$, $N(C_2H_5)_3$, $NH(CH_3)_2$, $NH(C_3H_7\text{-}n)_2$, $N(Benzyl)(C_6H_{13})_2$, $NH(C_3H_7\text{-}i)_2$, $NH_2(C_3H_7\text{-}i)$, $NH_2(CH_3)$, $NH_2(C_4H_9\text{-}n)$ $NH_2(Cyclohexyl)$, $NH_2(C_6H_5)$, $NH_2CH_2CH_2C_6H_5$, $NH_2CH(C_6H_5)CH_3$, $^{\oplus}N(CH_3)_4$, $^{\oplus}N(CH_3)_3(Benzyl)$ usw.

Die Verbindungen der Formel I sind stabile Substanzen, die sehr gute mikrobizide Eigenschaften aufweisen und sich gegen phytopathogene Pilze einsetzen lassen. Zusammen mit geeigneten, in der Agrarchemie üblichen Trägerstoffen lassen sich daraus wirksame Schädlingsbekämpfungsmittel herstellen.

Eine bevorzugte Gruppe von Mikrobiziden besteht aus Verbindungen der Formel I, worin $R_1$ Wasserstoff oder $C_1$-$C_3$-Alkyl bedeutet und $R_2$ Wasserstoff, $C_1$-$C_4$-Alkyl oder einen unsubstituierten oder durch Kohlenwasserstoffreste mit maximal 20 C-Atomen substituierten Ammoniumrest oder das Metallionenäquivalent eines, zweier oder mehrerer Metall-Kationen darstellt. Diese sollen Verbindungsgruppe Ia genannt werden.

Unter diesen sind solche bevorzugt, worin $R_1$ Wasserstoff, Methyl, Ethyl oder Isopropyl bedeutet und $R_2$ Wasserstoff, Methyl, Ethyl, Propyl, Isopropyl oder einen unsubstituierten oder substituierten Ammoniumrest darstellt, dessen Substituenten Wasserstoff, Alkylgruppen und 0 bis 2 Phenylgruppen und/oder Benzylgruppen sind, oder das Metallionenäquivalent von Li, Na, K, Mg, Ca, Cu, Mn, Zn, Sn, Fe, Ni, Co, Cr, Al, Ti, Zr oder von zweien oder von mehreren dieser Elemente bedeutet. (= Untergruppe Ib). Unter diesen sind solche besonders bevorzugt, worin $R_1$ Wasserstoff bedeutet (= Untergruppe Ic), und unter diesen wiederum jene worin $R_2$ Was-

serstoff oder ein, zwei oder mehrere der genannten Metallionenäquivalente darstellt (= Untergruppe Id), vor allem solche, worin $R_2$ Wasserstoff oder ein Metallionenäquivalent von Na, K, Mg, Ca, Cu, Mn, Zn, Fe, Ni oder Al darstellt (= Untergruppe Ie).

Eine weitere wichtige Verbindungsgruppe stellen auch solche innerhalb des Umfangs der Untergruppe Ib dar, worin $R_1$ Wasserstoff bedeutet und $R_2$ Wasserstoff oder $NH_4^+$ ist, dessen Wasserstoffatome auch ganz oder teilweise durch 0 bis 1 Phenyl oder 0 bis 1 Benzyl und 4 bis 1 Alkylgruppen mit maximal 4 C-Atomen ersetzt sein können (= Untergruppe If), insbesondere solche, bei denen die Alkylgruppen ausgewählt sind aus Methyl, Ethyl, Propyl, Isopropyl, Butyl und tert.Butyl (= Untergruppe Ig).

Wichtige Einzelverbindungen sind z.B.
1-Amino-cyclohexancarbonsäure,
1-Amino-3-methylcyclohexancarbonsäure
sowie ihre Monoalkylammoniumsalze.

Die vorliegende Erfindung betrifft entsprechend auch Verfahren zum Schützen von Pflanzen vor Pilzbefall, indem man die Pflanze, ihre Pflanzenteile oder den Ort ihres Wachstums mit einer fungizid wirksamen Menge eines 1-Aminocyclohexancarbonsäure-Derivats der Formel I bzw. Ia bis Ig behandelt. Ein wichtiges Einsatzgebiet ist dabei die Behandlung des Vermehrungsguts zum Schutz vor Mikroorganismen, insbesondere des Saatguts, wobei auch solche Verfahren eingeschlossen sind, bei denen behandeltes oder auch unbehandeltes Saatgut in einen mit Verbindungen der Formel I vorbehandelten Nährboden gegeben wird, beispielsweise in die Ackerfurche ("Drench-Applikation").

Die Erfindung bezieht sich gleichermassen auf so gebeiztes Pflanzenvermehrungsgut, insbesondere auf gebeiztes Saatgut.

1-Aminocyclohexancarbonsäure-Derivate der Formel I sind zum Teil bekannt.

So werden beispielsweise die 1-Aminocyclohexancarbonsäure selbst und Analoge in der US-PS-3,746,495 als Pharmazeutika des Gastrointestinal-Trakts zur Behandlung von Geschwüren vorgeschlagen.

In der JP-Auslegeschrift Nr. 731195 8 werden 1-Aminocyclohexancarbonsäure und ihr Na-, K-, Ca-, Mg- oder Sulfaminsäure-Salz als ungiftiger und dem Saccharin und Cyclamat in der Süsskraft überlegener Süssstoff vorgeschlagen. Präparate der Formel I sind also für Warmblüter völlig unbedenklich und stellen daher selbst bei unsachgemässer Anwendung oder nachlässiger Aufbewahrung keine Gefahr für den Endverbraucher oder für unbeteiligte Personen (z.B. Kinder) dar.

Verbindungen der Formel I lassen sich leicht nach der Strecker-Synthese zur Gewinnung von Aminosäuren herstellen. [Houben-Weyl, "Methoden der organ. Chemie XI/2, 269 (1958)]

Durch Addition von wässriger Blausäure oder von Cyanid an die Ketimin-Doppelbindung des intermediär gebildeten Derivats der Formel III

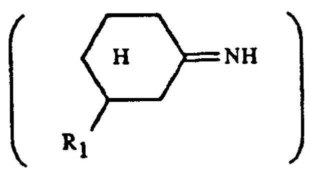

(III),

das durch Reaktion des entsprechenden Cyclohexanon-Derivats in Gegenwart von Ammoniak entsteht, und Verseifung des gebildeten 1-Cyano-cyclohexylamins der Formel IV

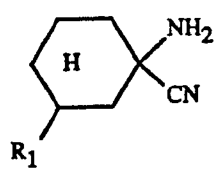

(IV)

werden die Grundkörper erhalten, die auf übliche Art, sofern gewünscht, in die entsprechenden Ester, Salze oder Metallkomplexe der Formel I überführt werden können.

Nach einer weiteren Methode lassen sich diese Grundkörper der Formel I auch nach der Bucherer-Hydantoinsynthese unter Hochdruck (ca. 5-20 atü) wie folgt herstellen :

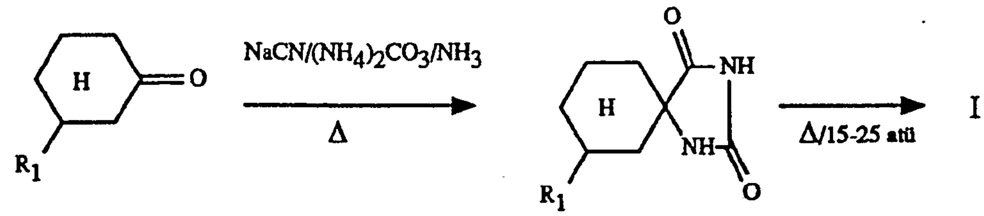


V

Dabei werden die entsprechenden Cyclohexanon-Derivate im Autoklaven bei erhöhter Temperatur mit Alkalicyanid und Ammoniumcarbonat in ammoniakalischer Lösung zu Hydantoin-Derivaten der Formel V umgesetzt, aus denen anschliessend durch alkalische oder saure Hydrolyse die 1-Aminocylohexancarbonsäure-Derivate der Formel I gewonnen werden [H.T. Bucherer, U.A. Lieb, J. prakt. Chemie, 141, 5 (1934)].

Die Bildung von Zwischenprodukten der Formel V kann bei 5 bis 20 atü, vorzugsweise 8 bis 12 atü, und Temperaturen von 80° bis 150°C erfolgen.

Es kann sich als zweckmässig erweisen die Hydrolyse von Verbindungen der Formel V ebenfalls im Autoklaven durchzuführen. Man arbeitet üblicherweise in Gegenwart eines Alkalihydroxids, z.B. Natriumhydroxid oder Kaliumhydroxid, bei Temperaturen von 150° bis 220°C und Drucken von 15 bis 25 atü, vorzugsweise 17 bis 20 atü.

Die Reaktion kann analog zu bereits bekannten Synthesen cyclischer Aminocarbonsäuren durchgeführt werden, beispielsweise analog zu Journal of Medicinal Chemistry Vol. 16, No. 7, 823 (1973).

Es wurde nun überraschend gefunden, dass 1-Aminocyclohexancarbonsäure-Derivate der Formel I ein für praktische Bedürfnisse des Pflanzenschutzes sehr günstiges Mikrobizid-Spektrum aufweisen. Ihr Haupteinsatzgebiet liegt in der Bekämpfung von schädlichen Mikroorganismen, vor allem von phytopathogenen Pilzen. So besitzen die Verbindungen der Formel I sehr günstige kurative, präventive und systemische Wirkung zum Schutz von Kulturpflanzen, ohne diese durch unerwünschte Nebenwirkungen zu beeinflussen. Kulturpflanzen seien im Rahmen vorliegender Erfindung beispielsweise : Getreide (Weizen, Gerste, Roggen, Hafer, Reis) ; Rüben : (Zucker- und Futterrüben) ; Kern-, Stein- und Beerenobst : (Aepfel, Birnen, Pflaumen, Pfirsiche, Mandeln, Kirschen, Erdbeeren, Himbeeren und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind gegen die den folgenden Klassen angehörenden phytopathogenen Pilze wirksam : Ascomycetes (z.B. Venturia) ; Basidiomycetes (z.B. Puccinia, Rhizoctonia) ; Fungi imperfecti (z.B. Cercospora) ; insbesondere gegen die zur Klasse der Phycomycetes gehörenden Oomyceten wie Phytophthora, Bremia, Pseudoperronospora, Plasmopara oder z.B. Pythium. Ausserdem wirken die Verbindungen der Formel I auch gegen schädliche Bakterien, so z.B. gegen die zur Klasse der Pseudomonadaceae gehörenden Xanthomonas Arten, Pseudomonas aeruginosa sowie gegen weitere ubiquitäre Bakterien wie z.B. Corynebacterium, Eschericia coli, Streptococcus- und Staphylococcus-Spezies.

Verbindungen der Formel I wirken systemisch. Daneben können sie mit sehr gutem Erfolg als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die neuen Verbindungen im Umfang der Formel I sowie mikrobizide Mittel, die solche Verbindungen als Wirkstoffe enthalten.

Unter den niedermolekularen Estern sind dies folgende Verbindungen :

1-Amino-cyclohexancarbonsäure-sek.butylester,
1-Amino-cyclohexancarbonsäure-isobutylester,
1-Amino-3-methylcyclohexancarbonsäure-ethylester,
1-Amino-3-methylcyclohexancarbonsäure-n-propylester,
1-Amino-3-methylcyclohexancarbonsäure-n-butylester,
1-Amino-3-methylcyclohexancarbonsäure-sek.butylester,
1-Amino-3-methylcyclohexancarbonsäure-isobutylester,

1-Amino-3-methylcyclohexancarbonsäure-tert.butylester.

Die Verbindungen der Formel I zeichnen sich bei den im Pflanzenschutz üblichen und selbst bei höheren Aufwandmengen durch auffallend gute Pflanzenverträglichkeit aus, so dass sie Kulturpflanzen vor schädlichen Mikroorganismen schützen, ohne sie in der Entwicklung zu beeinträchtigen.

Zur Bekämpfung dieser Mikroorganismen können die Verbindungen der Formel I für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Wirkstoffe der Formel I können auch im Gemisch mit anderen pestiziden oder pflanzenwuchsverbessernden Präparaten verwendet werden. Sie können auch selbst in Form entsprechender Metallkomplexe bei Erdbodenapplikation gleichzeitig als Spurenelementvermittler dienen.

Die Verbindungen der Formel I werden dabei zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage : Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctyl- phthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit.

Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkyrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Der Gehalt an Wirkstoff in handelsfähigen Mitteln liegt zwischen 0,1 bis 90 Gewichtsprozent, bevorzugt bei 2 bis 80 Gewichtsprozent, wobei der Verbraucher eher verdünnte Mittel anwendet.

In den nachfolgenden Beispielen sind Temperaturen in Celsiusgraden angegeben. Teile und Prozentangaben beziehen sich auf das Gewicht.

Herstellungsbeispiele

a) Herstellung des Zwischenproduktes

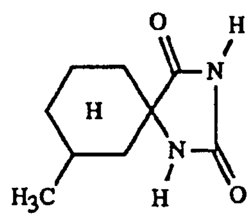

2-(3'-Methylcyclohexyl)-tetrahydroimidazolyl-3,5-dion

Ein Gemisch aus 1 gMol 3-Methylcyclohexanon, 2,2 gMolen Natriumcyanid, 1,5 gMolen Ammoniumcarbonat, 2 Litern konzentrierter wässriger Ammoniak-Lösung und 2 Litern Ethanol werden unter Rühren im Autoklaven 3 Stunden auf 120°C erhitzt, wobei der Innendruck auf 12 atü ansteigt. Anschliessend wird das Ethanol abdestilliert, das Reaktionsgemsich auf Raumtemperatur abgekühlt und filtriert. Der Rückstand wird mit Wasser salzfrei gewaschen und über Phosphorpentoxid getrocknet.
Ausbeute : 86 % (hellgelbe Kristalle).

Elementaranalyse (in %):

| | C | H | N |
|---|---|---|---|
| berechnet: | 59,33 | 7,75 | 15,38 |
| gefunden: | 58,9 | 7,6 | 15,1 |

b) Herstellung des Endproduktes

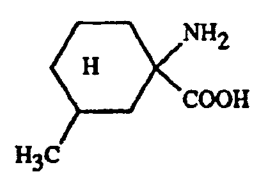

(6)

1-Amino-3-methylcyclohexancarbonsäure

1 gMol des nach a) hergestellten 2-(3'-Methylcyclohexyl)-tetrahydroimidazolyl-3,5-dions werden in 3,9 Litern 1,25 normaler wässriger Natronlauge im Autoklaven unter Rühren 2 Stunden lang auf 195°C erhitzt ; hierbei steigt der Innendruck auf 20 atü. Anschliessend wird die Reaktionslösung auf Raumtemperatur abgekühlt, mit 2 Litern Wasser verdünnt und mit Chlorwasserstoffsäure auf einen pH-Wert von ca. 1-2 eingestellt, wobei geringe harzige Ausflockungen auftreten, die abfiltriert werden. Das Filtrat wird nun mit wässriger Natronlauge auf einen pH-Wert von 6-7 eingestellt, auf 1/3 des Volumens eingeengt und mit Eiswasser gekühlt. Das Rohprodukt wird abfiltriert und portionsweise mit kaltem Wasser salzfrei gewaschen. Ausbeute 92 %. (farblose Kristalle)

Elementaranalyse (in %):

| | C | H | N |
|---|---|---|---|
| berechnet: | 61,1 | 9,6 | 8,9 |
| gefunden: | 61,4 | 9,5 | 8,3 |

Beispiel 2 : Herstellung des Metallkomplexes

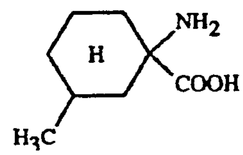 x $Cu^{++}$

Kupferkomplexe der 1-Aminocyclohexancarbonsäure

1 Teil 1-Amino-cyclohexancarbonsäure wird in 20 Teilen Wasser gelöst. Dazu werden 3 Teile Kupfer(II)acetat in Form einer 10%igen wässrigen Lösung zugetropft. Das Gemisch wird ca. 4 Tage bei Raumtemperatur gerührt. Der ausgeschiedene Kupferkomplex wird abfiltriert, mit wenig Wasser gewaschen und getrocknet. Ausbeute 70 % der Theorie.

Beispiel 3 : Herstellung von

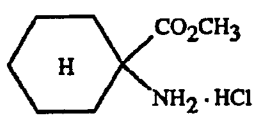 (24)

1-Amino-cyclohexancarbonsäuremethylester

1 g Aminocyclohexancarbonsäure wird in 20 ml Methanol suspendiert. Während 1 Minute wird HCl-Gas eingeleitet, wobei die Temperatur auf 40°C ansteigt. Die nun klare Lösung wird über Nacht unter Rückfluss gekocht. Anschliessend wird das Methanol unter vermindertem Druck entfernt, den Feststoff versetzt man nochmals mit 10 ml Methanol und befreit erneut vom Lösungmittel unter vermindertem Druck. Der Feststoff wird mit Aether digeriert (2 x 10 ml) und getrocknet. Man erhält die Titelverbindung mit einem Schmelzpunkt von 206-207°C.

Auf analoge Weise oder nach einer der weiter oben gegebenen Methoden lassen sich auch die folgenden Verbindungen der Formel I herstellen.

Tabelle 1

| Verb. Nr. | $R_1$ | $R_2$ | Komplex bzw. Salz | Elementaranalyse ber./gefunden | $^1$H-NMR (δ) oder andere physik. Konst. |
|---|---|---|---|---|---|
| 1 | H | H | -- | C: 58,7 / 57,5 %<br>H: 9,1 / 8,9 %<br>N: 9,8 /9,6 % | Smp. >250°C |
| 2 | H | H | $H_2SO_4$ | | |
| 3 | H | H | Zn | | Smp. >200°C |
| 4 | H | H | $H_2N$-$C_2H_5$ | | |
| 5 | H | H | Ca | | |
| 6 | $CH_3$ | H | - - | C: 61,1 / 61,4%<br>H: 9,6 / 9,5 %<br>N: 8,9 / 8,3 % | Lösungsm.: $CD_3OD$<br>$CH_3$: 0,9 (d, 5Hz, 3H)<br>Cyclo $C_6H_9$: 0,85-2,3 (m, 9H)<br>$H_2O$, $NH_2$: 4,87 |
| 7 | $CH_3$ | H | $H_2N$-iso$C_3H_7$ | -- | Lösungsm.: $D_2O$<br>$CH_3$: 1,2 (d, $J_{CH}$6Hz, 3H)<br>cyclo $C_6H_9$: 1,1-2,5 (m, 9H)<br>$C(CH_3)_2$: 1,62 (d, $J_{CH}$6Hz, 6H)<br>$H_2O$, $NH_2$: 4,96 |
| 8 | $CH_3$ | H | Cu | C: 43,5 / 43,2%<br>H: 6,8 / 7,1 %<br>N: 6,3 /6,0 %<br>Cu: 28,7 / 28,5 % | |
| 9 | H | $C_2H_5$ | -- | | $n_D^{21}$= 1.4606 |
| 10 | H | $CH_3$ | -- | | Sdp. 60-64°/1 mbar |
| 11 | $C_2H_5$ | $CH_3$ | $HNO_3$ | | |
| 12 | tert.$C_4H_9$ | H | -- | | |
| 13 | H | iso$C_3H_7$ | -- | | Sdp. 55-58°/0,07 mbar |

Tabelle 1 (Fortsetzung)

| Verb. Nr. | $R_1$ | $R_2$ | Komplex bzw. Salz | Elementar-analyse ber./gefunden | $^1$H-NMR (δ) oder andere physik. Konst. |
|---|---|---|---|---|---|
| 14 | H | Benzyl | -- | | |
| 15 | H | $nC_6H_{13}$ | -- | | |
| 16 | H | H | Na/K | | Smp. >250°C |
| 17 | H | H | Cu/Ni/Mn | | Smp. >200°C |
| 18 | H | H | Fe | | Smp. >280°C |
| 19 | H | H | $H_2N$-Benzyl | | |
| 20 | H | H | $N(CH_3)_2$Phenyl | | Smp. >180°C |
| 21 | H | H | $N(C_2H_5)_2$Benzyl | | Smp. >180°C |
| 22 | H | H | $H_2N$-$C_{12}H_{23}$n | | |
| 23 | H | H | Mn | | Smp. >250°C |
| 24 | H | $CH_3$ | HCl | | Smp. 206-207°C |
| 25 | $CH_3$ | $CH_3$ | HCl | | Smp. 190-200°C |

<u>Formulierungsbeispiele für Wirkstoffe der Formel I (%=Gewichtsprozent)</u>

| 2.1. Spritzpulver | a) | b) | c) |
|---|---|---|---|
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

2.2. Emulsions-Konzentrat

| | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

2.4. Extruder Granulat

| | |
|---|---|
| Wirkstoff aus der Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff aus der Tabelle 1 | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6. Suspensions-Konzentrat

| | |
|---|---|
| Wirkstoff aus der Tabelle 1 | 40 % |
| Ethylenglykol | 10 % |
| Nonylphenolpolyethylenglykolether (15 Mol) Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 1 : Wirkung gegen Phytophthora infestans auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Im Vergleich zu unbehandelten Kontrollpflanzen (100 % Befall) zeigten Pflanzen, die mit einer der Verbindungen Nr. 1, 3, 6, 7, 8, 10, 13, 16, 17, 18, 23, 24 oder 25 behandelt worden waren, weniger als 10 % oder keinen Befall.

b) Residual-kurative Wirkung

Tomatenpflanzen werden nach 3-wöchiger Anzucht mit einer Sporangiensuspension des Pilzes infiziert. Nach einer Inkubation von 22 Stunden in einer Feuchtkammer bei 90-100 % relativer Luftfeuchtigkeit und 20°C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 5 Tage nach der Infektion.

Tomatenpflanzen, die mit einer Spritzbrühe behandelt worden waren enthaltend eine der Verbindungen Nr. 1, 6, 7, 8, 10, 13, 17, 18 zeigten im Vergleich zu unbehandelten Kontrollpflanzen (100 % Befall) weniger als 10 % Pilzbefall.

c) Systemische Wirkung

Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Unter anderen zeigten in obigem Versuch folgende Verbindungen eine sehr gute systemische Wirkung : Nr. 1, 3, 6, 7, 8, 10, 13, 16, 17, 18, 20, 21 oder 23. Gegenüber unbehandelten Kontrollpflanzen (100 % Befall) bewirkten diese Verbindungen eine vollständige Unterdrückung des Pilzbefalls.

Beispiel 2 : Wirkung gegen Schimmelpilz an Feucht-Mais

Trockene Maiskörner (Portionen à 80 g) werden in verschliessbaren Plastikbechern mit der Prüfsubstanz in Form einer wässrigen Suspension, Emulsion oder Lösung gut durchmischt. Die Substanzapplikation wird so bemessen, dass eine Konzentration von 0,06 % AS bezüglich des Maistrockengewichts erreicht wird. Ein befeuchteter Papierlappen sorgt für eine feuchtigkeitsgesättigte Atmosphäre in den mit Mais gefüllten und anschliessend verschlosssenen Bechern. Nach 2-3 Wochen Inkubation bei ca. 20°C entwickelt sich bei den nur mit Wasser behandelten Mais-Proben spontan eine Mischpopulation von Schimmelpilzen. Eine künstliche Infektion erübrigt sich. Die Hemmung der Pilzentwicklung nach 3 Wochen dient zur Bewertung der Wirksamkeit der Prüfsubstanz.

Im Vergleich zu unbehandelten Maiskörnern (Kontrolle mit 100 % Befall) zeigten Körner, die mit Verbindungen der Formel I behandelt waren, eine starke Hemmung des Pilz-Befalls. Die Verbindungen Nr. 1 und 7 unterdrückten die Entwicklung der Schimmelpilze sogar vollständig.

## Patentansprüche

1. Mittel zum Bekämpfen von pflanzenpathogenen Pilzen und zur Verhütung von Pilzbefall, dadurch gekennzeichnet, dass sie als Wirkstoff mindestens ein 1-Aminocyclohexancarbonsäure-Derivat der Formel I

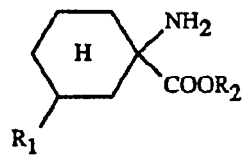

(I)

oder dessen Säureadditionssalze oder einen Metallkomplex von diesem enthalten, wobei $R_1$ Wasserstoff oder $C_1$-$C_4$-Alkyl bedeutet, und $R_2$ Wasserstoff, $C_1$-$C_6$-Alkyl, Benzyl, oder einen unsubstituierten oder durch Kohlenwasserstoffreste mit maximal 20 C-Atomen substituierten Ammoniumrest oder ein Metallionenäquivalent darstellt, zusammen mit einem pflanzenphysiologisch unbedenklichen Trägermaterial.

2. Mittel gemäss Anspruch 1 enthaltend eine Verbindung der Formel I, worin $R_1$ Wasserstoff oder $C_1$-$C_3$-Alkyl bedeutet und $R_2$ Wasserstoff, $C_1$-$C_4$-Alkyl oder einen unsubstituierten oder durch Kohlenwasserstoffreste mit maximal 20 C-Atomen substituierten Ammoniumrest oder das Metallionenäquivalent eines, zweier oder mehrerer Metall-Kationen darstellt.

3. Mittel gemäss Anspruch 2, enthaltend eine Verbindung der Formel I, worin $R_1$ Wasserstoff, Methyl, Ethyl oder Isopropyl bedeutet und $R_2$ Wasserstoff, Methyl, Ethyl, Propyl, Isopropyl oder einen unsubstituierten oder substituierten Ammoniumrest darstellt, dessen Substituenten Wasserstoff, Alkylgruppen und 0 bis 2-Phenylgruppen und/oder Benzylgruppen sind, oder das Metallionenäquivalent von Li, Na, K, Mg, Ca, Cu, Mn, Zn, Sn, Fe, Ni, Co, Cr, Al, Ti, Zr oder von zweien oder von mehreren dieser Elemente bedeutet.

4. Mittel gemäss Anspruch 3, enthaltend eine Verbindung der Formel I, worin $R_1$ Wasserstoff bedeutet.

5. Mittel gemäss Anspruch 4, enthaltend eine Verbindung der Formel I, worin $R_2$ Wasserstoff oder ein, zwei oder mehrere der genannten Metallionenäquivalente darstellt.

6. Mittel gemäss Anspruch 3, enthaltend eine Verbindung der Formel I, worin $R_1$ Wasserstoff bedeutet und $R_2$ Wasserstoff oder aber Ammonium ($=NH_4^+$) ist, dessen Wasserstoffatome auch ganz oder teilweise durch 0 bis 1 Phenyl oder 0 bis 1 Benzyl und 4 bis 1 Alkylgruppen mit maximal 4 C-Atomen ersetzt sein können.

7. Verfahren zum Schützen von Pflanzen vor Pilzbefall, dadurch gekennzeichnet, dass die Pflanze, ihre Pflanzenteile oder der Ort ihres Wachstums mit einer fungizid wirksamen Menge eines 1-Aminocyclohexancarbonsäure-Derivats der Formel I gemäss Anspruch 1 behandelt wird.

8. Verfahren gemäss Anspruch 7, dadurch gekennzeichnet, dass das Vermehrungsgut der Pflanze behandelt wird.

9. Verfahren gemäss Anspruch 7, wobei Saatgut in einen mit dem Wirkstoff der Formel I vorbehandelten Nährboden gegeben wird.

10. Pflanzenvermehrungsgut, das zum Schutz vor Pilzbefall mit einem 1-Aminocyclohexancarbonsäurederivat der Formel I gemäss Anspruch 1 gebeizt ist.

Europäisches Patentamt

# EUROPÄISCHER RECHERCHENBERICHT

Nummer der Anmeldung

EP 91 81 0029

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | JOURNAL OF THE CHEMICAL SOCIETY, 1961, Seiten 4372-4379, Londen, GB; L. MUNDAY: "Amino-acids of the cyclohexane series. Part I" * Insgesamt * --- | 1-10 | A 01 N 37/44<br>C 07 C 229/48<br>C 07 C 229/76 |
| A | CHEMICAL ABSTRACTS, Band 55, Nr. 3, 6. Februar 1961, Spalte 3004f, Columbus, Ohio, US; B. CIOCCA et al.: "Cutaneous action of some new synthetic amino acids", & PARFUM. COSMET. SAVONS 3, 363-8(1960) * Zusammenfassung * --- | 1-10 | |
| A | CHEMICAL ABSTRACTS, Band 96, Nr. 21, 24. Mai 1982, Seite 747, Zusammenfassung Nr. 181593w, Columbus, Ohio, US; A.M. EL-NAGGAR et al.: "Synthesis of some substituted 3-(2-thienyl)acryloyl amino acid derivatives", & GLAS. HEM. DRUS. BEOGRAD 1981, 46(1), 545-50 * Zusammenfassung * --- | 1-10 | RECHERCHIERTE SACHGEBIETE (Int. Cl.5) |
| A | CHEMICAL ABSTRACTS, Band 76, Nr. 1, 3. Januar 1972, Seite 305, Zusammenfassung Nr. 3429b, Columbus, Ohio, US; V. RUZICKA et al.: "Synthesis of some alpha-amino and alpha-nitrosocyclohexane-carboxylic acid esters", & SB. VYS. SK. CHEM.-TECHNOL. PRAZE, ORG. CHEM. TECHNOL. 1970, NO. 15, 127-131 * Zusammefnassung * --- -/- | 1-10 | A 01 N<br>C 07 C |

Der vorliegende Recherchenbericht wurde für alle Patentansprüche erstellt

| Recherchenort | Abschlußdatum der Recherche | Prüfer |
|---|---|---|
| DEN HAAG | 08-05-1991 | MUELLNERS W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Seite 2

Europäisches Patentamt

# EUROPÄISCHER RECHERCHENBERICHT

Nummer der Anmeldung

EP 91 81 0029

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF THE CHEMICAL SOCIETY, 1965, Seiten 6239-6244, Londen, GB; G.W. KENNER et al.: "Peptides. Part XVIII. Derivatives of 1-aminocyclohexanecarboxylic acid" * Seite 6242, Absatz 4; Seite 6243, Absätze 3,4 * | 1-10 | |
| A | JOURNAL OF THE CHEMICAL SOCIETY, 1962, Seiten 3977-3980, Londen, GB; R.J.W. CREMLYN: "Some new alicyclic alpha-amino-acids" * Seite 3977 - Seite 3978, Absatz 4; Seite 3979, Absatz 3; Seite 3980, vorletzter und letzter Absatz * | 1-10 | |
| A | CHEMICAL ABSTRACTS, Band 72, Nr. 25. 22. Juni 1970, Seite 309, Zusammenfassung Nr. 132134t, Columbus, Ohio, US; R.J.W. CREMLYN et al.: "Synthesis of some 1-amino alicyclic carboxylic acids and their derivatives", & INDIAN J. CHEM. 1970, 8(3), 218-20 * Zusammenfassung * | 1-10 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 08-05-1991 | MUELLNERS W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)